# EUROPEAN PATENT APPLICATION

(11) **EP 1 707 565 A1**
(43) Date of publication of application: **04.10.2006**
(21) Application number: 06004920.2
(22) Date of filing: 10.03.2006
(51) Int. Cl.: C07D 403/10, A61K 31/4178

(54) **Losartan potassium crystalline form alpha**

(30) Priority: 01.04.2005 IT MI20050551
(71) Applicant: Dipharma S.p.A., 33036 Mereto di Tomba (Udine) (IT)
(72) Inventor: Ventimiglia, Gianpiero, 20092 Cinisello Balsamo (MI) (IT); Allegrini, Pietro, 20097 San Donato Milanese (MI) (IT)
(74) Representative: Minoja, Fabrizio

(57) **Abstract**

Losartan potassium crystalline Form α, a process for its preparation, a pharmaceutical composition therefrom, its use in therapy and in a purification process of losartan potassium.

## Description

The present invention relates to a novel Losartan potassium crystalline form, herein referred to as Form alpha (α), a process for its preparation, a pharmaceutical composition therefrom and its use in therapy.

### TECHNOLOGICAL BACKGROUND

The compound Losartan potassium is the 2-n-butyl-4-chloro-1-[[2'-(1H-tetrazol-5-yl)[1,1'-biphenyl]-4-yl]methyl]-1-H-imidazole-5-methanol potassium salt. Losartan potassium (US 5,138,069) is an angiotensin II (AII) inhibitor useful in the treatment of angiotensin-dependent hypertension. Losartan potassium is also useful in the treatment of hypercholesterolemia, as it decreases total cholesterol. Furthermore, the administration of Losartan potassium in combination with a diuretic enhances the anthypertensive effect, while keeping the antiatherosclerotic and hypocholesterolemizing activities.

US 5,608,075 discloses two polymorphic forms of Losartan potassium (Form I and II). US 2004/0097568 describes a further polymorphic form of Losartan potassium, referred to as Form III. WO 03/048135 discloses two further Losartan potassium solvate forms (Form IV and V), and a hydrated form. WO 2004/039352 claims Losartan potassium in the amorphous form.

### SUMMARY OF THE INVENTION

It has now been found that Losartan potassium can exist, in addition to the above mentioned crystalline forms, also in a novel crystalline form stable at room temperature, herein referred to as Form α. Therefore, the invention relates to said form, a process for its preparation and a pharmaceutical composition comprising excipients and, as the active ingredient, Losartan potassium Form α.

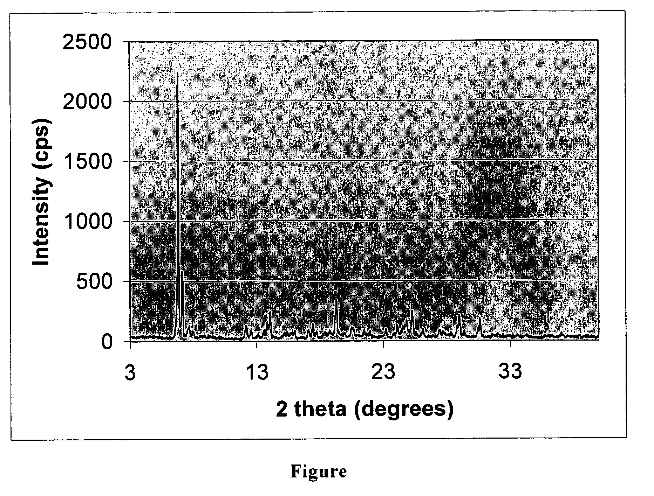

### BRIEF DISCLOSURE OF THE FIGURE

The figure shows the X ray diffraction spectra of Losartan potassium Form α (XRPD) recorded with an APD-2000 automated θ/θ diffractometer (Ital-Structures) under the following operative conditions: CuK radiation (λ = 1.5418 A), scanning angular range 0.03° for a time of 1 sec.

### DETAILED DISCLOSURE OF THE INVENTION

The first object of the present invention is Losartan potassium in the crystalline Form alpha (α). The XRPD spectrum reported in figure shows the more intense diffraction peaks at 6.75; 7.08; 12.15; 14.07; 17.46; 19.29; 24.09; 24.81; 25.26 and 29.01 ± 0.2 in 2 θ. Said Form α is prepared with a process comprising:
- preparation of a suspension of Losartan potassium in an anti-solvent;
- addition of a polar protic solvent to the above suspension, to form a dispersion in said solvent;
- crystallization of Losartan potassium Form α and recovery of the solid.

An anti-solvent is typically an aromatic solvent such as benzene; toluene; ortho-, meta- or para-xylene or mixtures thereof; anisole; chlorobenzene; preferably toluene or the xylenes, more preferably toluene. A polar protic solvent is for example a straight or branched C₁-C₆ alkanol, preferably a C₁-C₄ alkanol, typically selected from methanol, ethanol, 1-propanol, 2-propanol, 1-butanol, 2-butanol. Particularly preferred are methanol and ethanol, more preferably methanol.

The preparation of a Losartan potassium suspension in an anti-solvent can be carried out by contacting Losartan potassium, for instance Form I, with the anti-solvent. The amount of Losartan potassium in the starting suspension can approximately range from 5 to 30% w/w, preferably from 5 to 15%. The suspension in the anti-solvent is prepared with warming, typically at the reflux temperature. The resulting system is added with a polar protic solvent to obtaining a clear or opalescent dispersion which is cooled to a temperature preferably ranging from 15 to 20°C, thereby separating Losartan potassium Form α. Crystallization can optionally be promoted by seeding the dispersion with a compound having Losartan-related structure, such as a compound belonging to the "sartans" class, typically Valsartan, Olmesartan or Irbesartan, preferably Irbesartan Form A or Losartan potassium Form α itself, previously obtained according to the invention. The resulting product can be recovered by filtration or centrifugation, followed by washing with the same anti-solvent as used above and final drying, for example under vacuum. The drying temperature depends on the solvent used. Valsartan, Olmesartan and Irbesartan are known compounds, in particular Irbesartan Form A is disclosed in EP 454511.

As used herein, "about" and "approximately" mean around ± 10%.

The importance of the novel Losartan potassium crystalline form is, *inter alia,* mainly in its useful application in the pharmaceutical technique, in particular during the filtration, drying, sieving, formulation procedures and the like. Losartan potassium Form α of the invention can be used in human and veterinary medicine in the treatment of those conditions which can be treated with the known Losartan potassium crystalline forms, particularly in the treatment of hypertension and myocardial infarction. The treatment, as indicated above, can also be carried out in combination with other medicaments commonly used in the treatment of said pathologies.

A further object of the invention is a pharmaceutical composition comprising a pharmaceutically acceptable excipient and/or carrier and, as the active ingredient, Losartan potassium crystalline Form α, alone or in combination with at least one of the other known Losartan potassium forms. Typically, a combination with 1, 2 or 3 of the other known Losartan potassium polymorphic or amorphous forms described above may be used.

The daily dosage approximately ranges from 1 to 500 mg, preferably from 10 to 100 mg, in one or repeated administrations. Losartan potassium Form α can be formulated, according to known methods, in a suitable pharmaceutical form for the oral or parenteral use. Examples of said forms are solutions, suspensions and emulsions for the oral or parenteral use, tablets, pills, capsules, mixtures of powders, syrups and suppositories.

The process described above to obtain losartan potassium Form α allows to purify the final product from any impurities formed during the process for the synthesis of losartan potassium, deriving from side reactions and degradations of the product itself.

Accordingly, a further object of the invention is a process for the purification of losartan potassium, comprising the conversion of losartan potassium into losartan potassium Form α and, if desired, its subsequent conversion into another losartan potassium form according to known procedures, for instance the ones described in US 5,608,075, US 2004/0097568, WO 03/048135 and WO 2004/039352.

Losartan potassium starting material may be any losartan potassium, for instance the crude product as obtainable according to US 5,138,069 or the hydrated, amorphous or crystalline forms described above. A losartan potassium final product may be Form α or any of the known hydrated, amorphous or crystalline forms.

Said process affords losartan potassium Form α, Form I, Form II, Form III, Form IV, Form V, and the above hydrated and amorphous forms with a purity higher than 99.0%, in particular higher than 99.9%, i.e. suitable to fulfil the regulatory requirements for medicaments.

The following example illustrates the present invention.

### EXAMPLE 1

### Preparation of Losartan potassium Form α

5 g of Losartan potassium Form I are suspended in 50 ml of toluene. The suspension is refluxed under stirring and carefully dropwise added with 30 ml of methanol, until obtaining an opalescent dispersion. The system is seeded with Irbesartan Form α and the mixture is slowly cooled at room temperature, and kept under stirring for a time of about 12 hours, to allow losartan potassium Form α to crystallize. The product is recovered by filtration, washed with toluene (2 x 5 ml) and dried under vacuum at a temperature of 50°C. Approx. 3 g of Losartan potassium Form α are obtained, having an XRPD spectrum substantially as reported in the Figure.

### EXAMPLE 2

### Preparation of Losartan potassium Form α

5 g of losartan potassium, as obtained according to US 5,608,075, are suspended in 50 ml of para-xylene. The suspension is refluxed under stirring and carefully dropwise added with 30 ml of ethanol, until obtaining an opalescent dispersion. The system is seeded with irbesartan Form α and the mixture is slowly cooled at room temperature, and kept under stirring for a time of about 12 hours, to allow losartan potassium Form α to crystallize. The product is recovered by filtration, washed with para-xylene (2 x 5 ml) and dried under vacuum at a temperature of 50°C. Approx. 3.2 g of losartan potassium Form α are obtained, having an XRPD spectrum substantially as reported in the Figure.

## Claims

1. Losartan potassium crystalline Form alpha (α) wherein the more intense diffraction peaks are observed at 6.75; 7.08; 12.15; 14.07; 17.46; 19.29; 24.09; 24.81, 25.26 and 29.01 ± 0.2 in 2θ.

2. Crystalline form according to claim 1, having an XRPD spectrum substantially as illustrated in the Figure.

3. A process for the preparation of Losartan potassium in the crystalline form alpha, as defined in claim 1, comprising:
• preparation of a suspension of Losartan potassium in an anti-solvent;
• addition of a polar protic solvent to the above suspension, to form a dispersion in said solvent;
• crystallization of Losartan potassium Form α and recovery of the solid.

4. A process according to claim 3, wherein the anti-solvent is an aromatic solvent.

5. A process according to claim 3, wherein the Losartan potassium concentration in the suspension approximately ranges from 5 to 30%.

6. A process according to claim 3, wherein the polar aprotic solvent is added to the Losartan potassium suspension in an anti-solvent at the reflux temperature.

7. A process according to claim 3, wherein the polar protic solvent is a C₁-C₆ alkanol.

8. A process according to any one of claims 3 to 7, wherein the dispersion is seeded with a compound with Losartan-related structure.

9. A pharmaceutical composition comprising a pharmaceutically acceptable excipient and/or carrier and, as the active ingredient, Losartan potassium crystalline Form α, either alone or in combination with at least one of the known Losartan potassium forms.

10. A process for the purification of losartan potassium, comprising the conversion of losartan potassium into losartan potassium Form α, according to claim 3, and, if desired, its subsequent conversion into another losartan potassium form.
